# EUROPEAN PATENT SPECIFICATION

(11) **EP 2 377 475 B1**
(45) Date of publication and mention of the grant of the patent: **04.06.2014**
(21) Application number: 11161095.2
(22) Date of filing: 05.04.2011
(51) Int. Cl.: A61B 17/20, A61B 17/34, A61H 39/08, A61B 19/00

(54) **Apparatus for treating a tennis elbow**
Vorrichtung zur Behandlung eines Tennisellbogens
Appareil de traitement d'épicondylite latérale

(30) Priority: 15.04.2010 NL 2004558
(43) Date of publication of application: 19.10.2011
(73) Proprietor: ITEC Medical B.V., 3171 PN Poortugaal (NL)
(72) Inventor: Van Thiel, Robert Cornelis, 4847 NM Teteringen (NL)
(74) Representative: Nederlandsch Octrooibureau

(56) References cited:
- WO-A2-00/74763
- WO-A2-2007/002227
- WO-A2-2009/105628
- CH-A- 396 311
- DE-A1- 3 108 766
- US-A- 4 696 308
- US-A- 5 735 868
- US-A- 5 833 627
- US-A1- 2004 030 325
- US-A1- 2007 149 991
- US-A1- 2008 221 520
- US-A1- 2009 065 565
- US-A1- 2009 149 830
- US-B1- 6 551 338
- US-B1- 6 611 707
- US-B1- 6 702 805

## Description

The present invention relates to an apparatus for treating a tennis elbow, comprising: an apparatus body, a fixing part which is arranged near an outer side of the apparatus body for fixably positioning the apparatus body with respect to its surroundings, a part which is arranged in or near the apparatus body and can be extended with respect to the apparatus body, a positioning device which is arranged in or near the apparatus body for positioning the extendable part with respect to the apparatus body and a holder which is exchangeably connected to the extendable part during use, comprising a holder part with penetration means attached thereto, wherein the penetration means are arranged in a pattern on the holder part.

Approximately 1-3% of the general population is affected by a tennis elbow. In particular the age group between 35-54 years of age suffers from this complaint, with men and women both being affected to the same degree by this medical condition. Contrary to popular belief, a tennis elbow is not usually caused by playing tennis. This cause only accounts for 5-10% of patients.

It is known that a tennis elbow (medical term: epicondylitis lateralis) involves a degeneration of the tendon of the muscle known as "musculus extensor carpi radialis brevis" (ECRB).

The object of treating a tennis elbow is to alleviate the pain in the elbow and to return the elbow to its normal function as soon as possible. To this day, it is not known which type of treatment is most effective. The most commonly used treatments of a tennis elbow are: physiotherapy, a brace, injections of corticosteroids, immobilisation by putting the elbow in plaster, surgery during which cuts are made in the affected tendon, and percutaneous interventions. The latter treatment is discussed in more detail below.

In medical practice, it has been found that a treatment of the affected tendon in the case of a tennis elbow by means of various manual penetration operations of this tendon by means of a penetration means can be effective. Whether this so-called "percutaneous intervention" is more effective than surgery is not known, but it is less invasive which means that the patient can resume his/her activities after such an intervention much sooner than after surgery.

However, with a percutaneous intervention, it is a problem to determine the exact position of the needle with respect to the patient, in order to ensure that the correct tissue is pierced and a correct position of the penetration means with respect to the object to be treated is achieved. As yet, this treatment is therefore carried out by a surgeon in cooperation with a radiologist. The surgeon himself/herself has to determine whether the needle pierces the correct tissue and whether the correct position of the penetration means with respect to the object to be treated has been reached; the radiologist has to provide an image of the penetration to the surgeon.

In patent publication CH 396,311, an apparatus is described which is aimed at stimulating the blood circulation in the skin by pricking the skin with needles. More particularly, the publication describes an apparatus having an apparatus body, a fixing part to fix the apparatus with respect to the skin area to be treated, and a displaceable part to insert the needles into the skin, in which the needles are mounted on a holder which is fitted so as to be replaceable in the displaceable part. Furthermore, the apparatus comprises a handle in order to push the displaceable part comprising the needles in the direction of the skin. The displaceable part is in this case provided with a spring in order to actuate the sliding movement in the direction of the skin. In addition, the apparatus is provided with mechanical limiters to prevent the needles from penetrating too far into the skin.

However, a drawback of such an apparatus is the fact that the exchangeability of the holder entails risks regarding hygiene. Thus, for example, it is possible that the user forgets to clean the holder after use, and that the holder is subsequently used to treat another patient. This significantly increases the risk of, in particular, infections.

It is an object of the present invention to provide an apparatus for the treatment of a tennis elbow which prevents a holder placed in the apparatus during use being re-used again subsequently.

To this end, the apparatus according to the invention as claimed in claim 1 has the feature that the apparatus body or the extendable part is provided with a manipulating element which manipulates a feature of the holder in such a way during use that the holder is made unsuitable for re-use in the apparatus and the feature that the holder comprises 10-20 needles and the needles have a length of approximately 15 mm.

The result of providing the apparatus body or the extendable part with a manipulating element which manipulates a feature of the holder during use is that the manipulated feature of the holder can be used to prevent the holder from being reinstalled again in the apparatus and from being re-used in the apparatus.

In an embodiment, the apparatus is provided with a disabling element which blocks the apparatus if a holder has been installed which has been made unsuitable for re-use. The disabling element blocks the operation of the apparatus, for example by preventing the positioning device from operating and thus preventing the displaceable part from sliding out.

In a further embodiment, the manipulating element comprises a mechanical deforming means which engages with the holder during first use and at least partly deforms the latter. The deformation in this case has the object, for example, to deform the holder in such a way that said holder cannot be installed in the extendable part for a second use. By way of example, it is possible to provide a holder attachment part with a bore in which the holder is attached to the extendable part during first use by means of a locking pin which is provided in a securing recess in the extendable part. After the first use, the bore is deformed, so that the locking pin cannot bring about a connection between the extendable part and the holder for a second use, as a result of which the apparatus cannot be used.

Moreover, the mechanical deformation can serve to inform the user of the holder in a purely visual way about whether or not the holder has already been used. In this case, consideration may be given to a lug which is present on the holder and which is pushed in when the holder is first installed in the extendable part, thus forming an indication for the operator of the apparatus that first use has already taken place.

In another embodiment, the holder is provided with an electronic memory and the apparatus is provided with a readout device for reading the memory, in which the manipulating element comprises an electronic writing means for writing a value to the electronic memory during use. This value is read by the readout device and can in this case be used to determine whether the holder has already been used.

In a further embodiment, the apparatus is provided with an evaluation circuit for, during use, evaluating the memory value read out of the electronic memory. The evaluation circuit can then communicate the value to the disabling element: if, by way of example, the value corresponds to or exceeds a certain reference value, the operation of the apparatus is inhibited by the disabling element. It is also possible for the holder to be connected to the extendable part by means of an electromagnetically actuated connection, in which the evaluation circuit of the disabling element severs the connection if a second use of the holder is detected.

In an embodiment, the apparatus is provided with a sensor for determining the position of the penetration means with respect to the object to be treated, wherein the sensor can detect the position of the penetration means with respect to the object to be treated and communicates the position to the positioning device if a threshold value with regard to the position of the penetration means with respect to the object to be treated has been exceeded. In this case, the sensor acts as a kind of emergency stop for the positioning device, if the position of the penetration means with respect to the object to be treated exceeds a certain value during penetration. In this case, consideration may be given to the undesired penetration of certain tissue which is situated under the target tissue.

In a further embodiment, the sensor comprises a mechanical resistance meter which at least partly determines the position of the penetration means with respect to the object to be treated by means of measuring the mechanical resistance of the penetration means in the object to be penetrated, including a human arm. Since tissues have different mechanical properties, also including mechanical resistance against penetration, it is advantageous to provide the sensor acting as an emergency stop with a mechanical resistance meter which provides the positioning device with information regarding the mechanical resistance experienced by the penetration means. If, for example, a bone layer is reached, the mechanical resistance meter will record a relatively high mechanical resistance value, which may be a signal for the positioning device to initiate an emergency stop.

In a further embodiment, the positioning device is provided with a gear rack connected to the extendable part in the longitudinal direction, which engages with a gear wheel drive which is placed in the apparatus body and is connected to an operating member which can be operated by an operator. The gear rack and gear wheel drive ensure that the extendable part can be positioned relatively accurately with respect to the object to be treated, such as an arm, which is of great importance in order to achieve the desired penetration depth.

In yet another embodiment, a light source which can be directed at the holder is attached to the apparatus body or the extendable part. The light source can be used to illuminate the target area for the penetration. In addition, the light source can also be used to mark the target area, for example by means of the shadow which is produced on the target tissue by the light source by means of the holder. In this case, it is advantageous to attach several light sources, preferably four, to the apparatus, in which case the light sources correspond to the number of corners or sides of the holder part. An optional combination with, for example, a transparent holder part in which light emitted by the light source passes through the holder part and impinges on the target area is advantageous.

The invention furthermore relates to a holder as claimed in claim 10, suitable for exchangeable connection with the extendable part of an apparatus according to one of the preceding claims, provided on a holder part with penetration means attached thereto which are arranged in a pattern on the holder part, wherein a feature of the holder can be manipulated in such a manner during use by a manipulating element arranged on the apparatus body or the extendable part, that the holder is made unsuitable for re-use in the apparatus and wherein the holder comprises 10-20 needles and the needles have a length of approximately 15 mm.

In an embodiment, the holder can be at least partly mechanically deformed by, during use, engaging with a manipulating element which is installed on the extendable part and which comprises a mechanical deforming means. In this case, the deformation is, for example, aimed at deforming the holder in such a way that it cannot be placed in the extendable part for a second use. In this case, as has already been mentioned, it is possible to provide a holder attachment part with a bore in which the holder is attached to the extendable part during first use, by means of a locking pin which is arranged in a securing recess in the extendable part. After the first use, the bore is deformed, so that the locking pin cannot produce a connection between the extendable part and the holder for a second use, as a result of which the apparatus cannot be used. In addition, as has already been mentioned above, the mechanical deformation can serve to inform the user of the holder in a purely visual way about whether or not the holder has already been used. In this case, consideration may be given to a lug which is present on the holder and which is pushed in when the holder is first installed in the extendable part, thus forming an indication for the operator of the apparatus that first use has already taken place.

In a further embodiment, the holder comprises an electronic memory. The apparatus according to the invention is in this case provided with a readout device for reading the memory, as has already been described above, with the manipulating element comprising an electronic writing means for writing a value to the electronic memory during use. This value is read out by the readout device and can in this case be used to determine whether the holder has already been used.

In another embodiment, a holder is provided in which the length of at least a part of the penetration means near the circumference of the holder part is smaller than the length of the other penetration means situated on the holder part. As a result of the fact that the length of the penetration means, near the circumference of the holder part, is smaller than the length of the other means situated on the holder part, a more uniform penetration is achieved when a curved object, for example a curved arm in the case of the treatment of a tennis elbow, is situated under the apparatus.

In a further embodiment, the holder part is at least partly convex on the side facing the object to be treated. By using a partially convex shape on the side facing the object to be treated, the penetration means can be more readily positioned and it is likewise possible to achieve a more even penetration when a curved object is placed under the apparatus.

In an embodiment, the holder is provided with one or more penetration means which are provided with a liquid inlet duct. Thus, it is possible, if desired, to inject a liquid into the target tissue via the penetration means.

In addition, it is advantageous to provide the apparatus with or to connect it with an ultrasonic unit for ultrasonically recording the penetration. Using ultrasonic equipment prevents the patient from being exposed to a relatively high dose of radiation, as is the case, for example, with a CT scan.

Likewise, it is advantageous to connect the ultrasonic unit to a graphic display means which shows the penetration graphic recorded by the ultrasonic unit to the operator of the apparatus. Thus, the surgeon can see in a relatively simple manner whether the penetration is being carried out correctly.

It is also advantageous for the penetration means to comprise needles. As the invention also aims to achieve a certain degree of standardisation, it is advantageous to use needles as the penetration means, since needles are customary in the medical field for performing a penetration in the human body.

It is likewise advantageous if the holder which, in use, is installed in the apparatus and on which the needles are arranged, has a length of approximately 20 mm and a width of approximately 8 mm not according to the invention. In this way, a holder is produced having dimensions which are relatively optimum for the percutaneous treatment of a tennis elbow. It is also advantageous if the needles in this case have a length of approximately 15 mm. Thus, an optimum flexural stiffness is achieved in proportion to an optimum penetration length.

It is also advantageous if the holder comprises 10-20, preferably 15, needles arranged in 3 rows of 5 needles each. In this way, the natural shape of the affected tendon can be used in the treatment of a tennis elbow.

The invention will be explained in more detail with reference to an embodiment by means of figures, in which:
Fig. 1a diagrammatically shows a side view in cross section of an embodiment of the apparatus according to the invention;
Fig. 1b shows a detail view of an apparatus according to the invention having a mechanical deformer as manipulating element;
Fig. 1c shows a detail view of an apparatus according to the invention having a writing means as manipulating element;
Fig. 2a diagrammatically shows a perspective view of an embodiment of a holder according to the invention;
Fig. 2b diagrammatically shows a perspective view of another embodiment of a holder according to the invention having a memory chip;
Fig. 3 shows a detail view of a needle to be installed on the holder and having a liquid inlet duct;
Fig. 4a shows a further embodiment of a convex holder according to the invention; and
Fig. 4b shows a further embodiment of a holder according to the invention, in which the needles have a smaller length near the circumference.

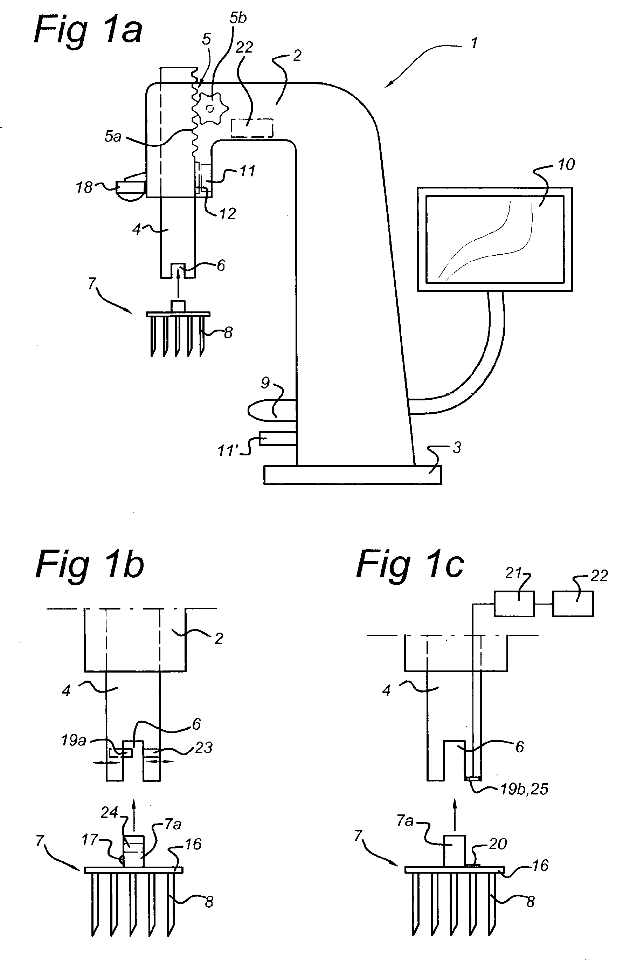

Fig. 1a shows a side view in cross section of an embodiment of the apparatus according to the invention. In this case, Fig. 1 shows the apparatus 1 with an apparatus body 2. The apparatus body 2 substantially closes off the internal parts of the apparatus 1 from its surroundings. In addition, Fig. 1 shows a fixing part 3 for fixably positioning the apparatus 1 with respect to its surroundings. The fixing part 3 may, for example, in an embodiment, be connected to a piece of furniture situated in the treatment space. Consideration may also be given to attaching the fixing part 3 to a wall of the treatment space. In the apparatus body 2, a sliding arm 4 is provided which is configured to undergo a translational movement on account of a positioning device 5. However, it is also conceivable for the sliding arm 4 to undergo a rotating movement on account of the positioning device 5. This may be desirable, for example, in case of a lack of space to move for the sliding arm 4, or in case the object to be treated, such as a human arm, is curved to such a degree that a rotating movement is more suitable.

In addition, Fig. 1a shows a recess 6 on the side of the sliding arm 4 which, in use, is the underside, which recess 6 can accommodate a holder 7 with needles 8 in an exchangeable manner. In the illustrated embodiment from Fig. 1a, the holder 7 has a protruding part which can be secured in the recess 6 of the sliding arm 4. However, it is conceivable for the recess 6 to be situated in the holder 7 and for the protruding part to be situated on the sliding arm 4. Of course, other similar methods of attachment are likewise possible. In the illustrated embodiment from Fig. 1a, the positioning device 5 comprises a gear wheel drive 5b connected to an actuating motor, in which the actuating motor, via the gear wheel drive 5b, drives a rack 5a which is fixedly attached to the sliding arm 4. However, other embodiments are conceivable, as long as it is ensured that the positioning device 5 has to be able to accurately position the sliding arm 4 with respect to the apparatus body 2.

Furthermore, the embodiment of the apparatus 1 illustrated in Fig. 1a comprises an ultrasonic unit 9 for, in use, ultrasonically recording the penetration. The recording of the penetration by the ultrasonic unit 9 is shown to the operator of the apparatus 1, usually the treating surgeon, by means of a graphic display means 10. Fig. 1 also shows a sensor 11 at a first position for determining the position of the needles 8 with respect to the object to be treated. The sensor 11 communicates the position of the needles with respect to the object to be treated to the positioning device 5 if a threshold value with regard to the position of the needles 8 with respect to the object to be treated is exceeded. In this case, the sensor 11 acts as a kind of emergency stop for the positioning device 5, if said position of the needles 8 with respect to the object to be treated exceeds a certain value during the penetration. This sensor 11 may be in communication with the ultrasonic unit 9 in order to achieve a certain degree of cooperation. The sensor 11 may also comprise a mechanical resistance meter 12 which at least partly determines the position of the needles 8 with respect to the object to be treated by measuring the mechanical resistance of the needles 8 as experienced in the object to be penetrated. In this case, it may be desirable to install a sensor 11' at a second position, near or against the sliding arm 4, so that the mechanical resistance measurement can be carried out with a high degree of accuracy. Obviously, other positions for the sensor 11 are also conceivable, such as on the holder 7 or in or near the recess 6. It is also not strictly necessary to install the sensor 11 on the apparatus 1, the sensor 11 can also be installed in the vicinity of the apparatus 1. This also applies to the sensor 11 as shown in the first position.

Fig. 1b shows a detail view of an apparatus 1 according to the invention having a mechanical deformer 19a as manipulating element. The holder 7 comprises an attachment part 7a which is fitted in the recess 6, a plate-shaped holder part 16 and needles 8. The plate-shaped holder part 16 may be made from an optionally non-translucent material. The attachment part 7a is provided with a bore 24. During first use, the holder 7 is connected to the sliding arm 4 as a result of the fact that a locking pin 23 is pushed from the sliding arm into the bore 24. After use, the bore 24 is deformed due to the fact that a deformer 19a crushes the bore 24, for example by means of a small actuated hammer which is comprised by the deformer 19a, as a result of which the locking pin 23 cannot be introduced into the bore 24. This prevents a connection between the holder 7 and the sliding arm 4 from being formed in the case of a second use, as a result of which the holder 7 will fall out of the apparatus 1 and the apparatus 1 cannot be used. Furthermore, Fig. 1b shows a lug 17 which can be used to visually determine whether the holder 7 has been used before. In this case, the lug 17 is, for example, arranged in such a manner on the attachment part 7a that the lug 17 is pushed in during installation of the former in the sliding arm 4.

Fig. 1c shows a detail view of an apparatus 1 according to the invention having a writing means 19b as manipulating element. In this case, the holder 7 is provided with a memory chip 20 and the apparatus 1 is provided with a readout device 25 for reading the memory chip 20 fitted on the holder part 16, with the writing means 19b writing a value to the memory chip 20 at a first use. This value is read out by the readout device 25 and is used to determine whether the holder 7 has already been used: the apparatus 1 is also provided with an evaluation circuit 21 for evaluating the memory value which is stored in the memory chip 20. The evaluation circuit 21 can then communicate the value to the disabling element 22: if the value, for example, corresponds to or exceeds a certain reference value, the operation of the apparatus is inhibited by the disabling element 22. As has already been mentioned, it is possible for the holder 7 to be connected to the sliding arm 4 by means of an electromagnetically actuated connection, with the evaluation circuit 21 or the disabling element 22 interrupting the connection if a second use of the holder 7 is detected.

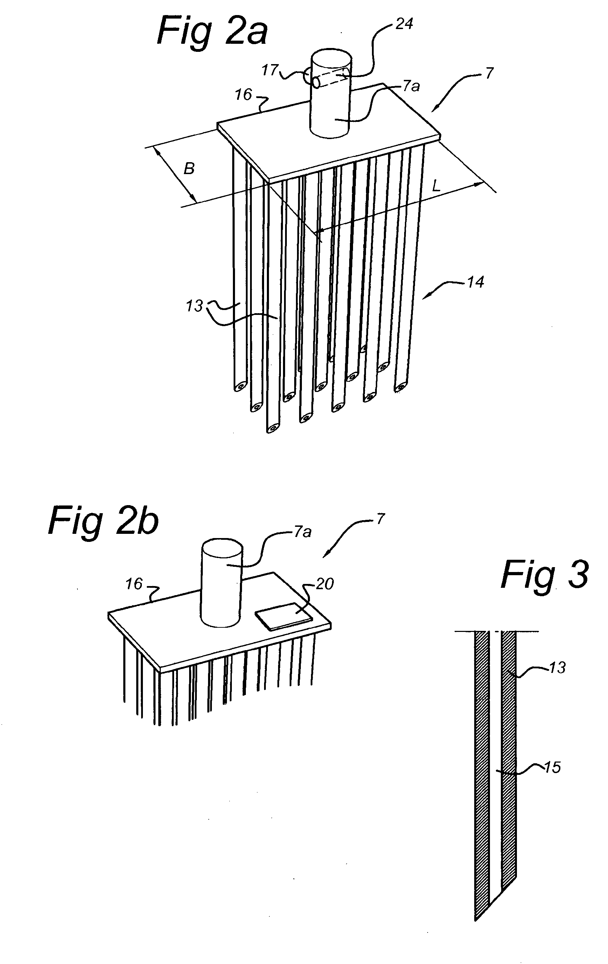

Fig. 2a shows a perspective view of an embodiment of a holder according to the invention. Fig. 2 in this case shows the holder 7 with a plate-shaped holder part 16, in which the needles 8 are arranged in rows. In the illustrated embodiment, the needles 8 comprise fifteen needles 13, arranged in three needle rows 14 of five needles. Of course, it is also conceivable for the needles 8 to only partly comprise needles 13, since it could also be desirable to combine other kinds of needles 8, for example small pins or tubes, with the needles 13, or with one another. Other patterns are, of course, also conceivable. The dimensions of the plate-shaped holder 16 are, for example, set to a length L of 20 mm and a width B of 8 mm, with the thickness being less important. The thickness is only important for making the holder 7 stiff. Obviously, it is also possible for the holder 7 to be given a different shape, such as a disc-shape. However, the main concern here is that the desired amount of needles 8 can be arranged on the holder 7, in the desired arrangement. Fig. 2a also shows the lug 17 on the holder attachment part 7a, in which case the lug 17 can be used to visually determine whether the holder 7 has already been used before.

Fig. 2b diagrammatically shows a perspective view of another embodiment of a holder 7 according to the invention with a memory chip 20 which is arranged on that side of the holder part 16 which faces the apparatus 1.

Fig. 3 shows a detail view of a needle 8 which is to be placed on the holder 7 and has an liquid inlet duct 15. The liquid inlet duct 15 is used to inject liquids into the object to be treated, such as an arm, via the needle 8.

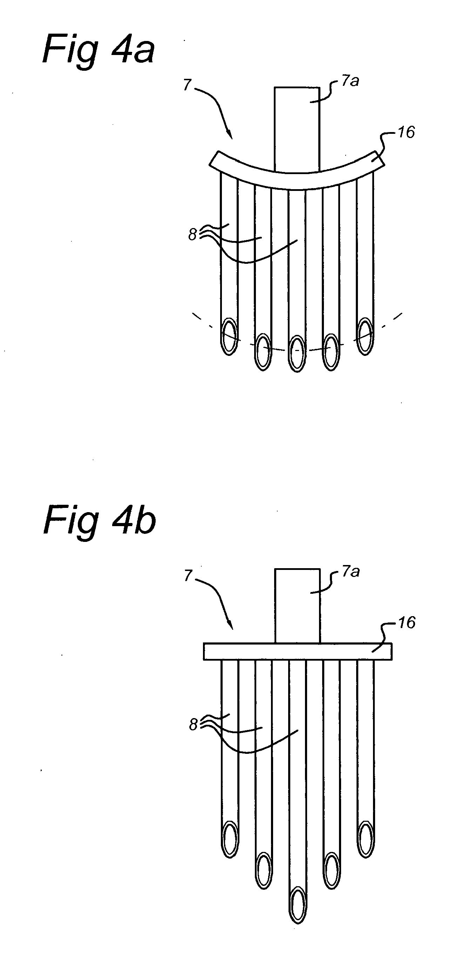

Fig. 4a shows a further embodiment of a convex holder 7 according to the invention. In Fig. 4a, the plate-shaped holder part 16 is convex in order to achieve a more controlled and uniform penetration if the object to be treated is curved.

Fig. 4b shows a further embodiment of a holder 7 according to the invention, in which the needles 8 have a smaller length near the circumference. In principle, this structure achieves the same effect as the structure illustrated in Fig. 4a. However, in this case, the plate-shaped holder part 16 retains its original rectangular shape.

### List of reference numerals

- 1.: Apparatus
- 2.: Apparatus body
- 3.: Fixing part
- 4.: Sliding arm
- 5.: Positioning device
- 5a.: Gear rack
- 5b.: Gear wheel drive
- 6.: Recess
- 7.: Holder
- 7a.: Holder attachment part
- 8.: Needles
- 9.: Ultrasonic unit
- 10.: Graphic display means
- 11.: Sensor
- 11'.: Sensor
- 12.: Mechanical resistance meter
- 13.: -
- 14.: Needle row
- 15.: Liquid inlet duct
- 16.: Plate-shaped holder part
- 17.: Lug
- 18.: Light source
- 19.: -
- 19a.: Mechanical deformer
- 19b.: Writing means
- 20.: Memory chip
- 21.: Evaluation circuit
- 22.: Disabling element
- 23.: Locking pin
- 24.: Bore
- 25.: Readout device

- L =: length of the plate-shaped holder part
- B =: width of the plate-shaped holder part

## Claims

1. Apparatus (1) for treating a tennis elbow by means of percutaneous intervention, comprising:
- an apparatus body (2),
- a fixing part (3) which is arranged near an outer side of the apparatus body (2) for fixably positioning the apparatus body (2) with respect to its surroundings,
- a part (4) which is arranged in or near the apparatus body (2) and can be extended with respect to the apparatus body (2),
- a positioning device (5) which is arranged in or near the apparatus body (2) for positioning the extendable part (4) with respect to the apparatus body (2),
- a holder (7) which is exchangeably connected to the extendable part (4) during use, comprising a holder part (16) with penetration means (8) attached thereto, comprising needles, wherein the penetration means (8) are arranged in a pattern on the holder part (16),
wherein the apparatus body (2) or the extendable part (4) is provided with a manipulating element (19a, 19b) which manipulates a feature of the holder (7) in such a way during use that the holder (7) is made unsuitable for re-use in the apparatus (1),
wherein the positioning device (5) is suitable for accurately positioning the extendable part (4) with respect to the apparatus body (2) to achieve the desired penetration depth for carrying out a percutaneous intervention of an elbow tendon, and wherein the holder comprises 10-20 needles and the needles have a length of approximately 15 mm.

2. Apparatus (1) according to Claim 1, furthermore provided with a disabling element (22) which blocks the apparatus (1) if a holder (7) has been installed which has been made unsuitable for re-use.

3. Apparatus (1) according to Claim 1 or 2, wherein the manipulating element (19a, 19b) comprises a mechanical deforming means which engages with the holder (7) during use and at least partially deforms the latter.

4. Apparatus (1) according to Claim 1 or 2, wherein the holder (7) is provided with an electronic memory (20) and the apparatus (1) is provided with a readout device (25) for reading the memory (20), wherein the manipulating element (19a, 19b) comprises an electronic writing means (19b) for writing a value to the electronic memory (20) during use.

5. Apparatus (1) according to Claim 4, wherein the apparatus (1) is provided with an evaluation circuit (21) for, during use, evaluating the memory value read out of the electronic memory (20).

6. Apparatus (1) according to one of the preceding claims, wherein the latter is provided with a sensor (11; 11') for determining the position of the penetration means (8) with respect to the object to be treated, wherein the sensor (11; 11') can detect the position of the penetration means (8) with respect to the object to be treated and communicates the position to the positioning device (5) if a threshold value with regard to the position of the penetration means (8) with respect to the object to be treated has been exceeded.

7. Apparatus (1) according to Claim 6, wherein the sensor (11; 11') comprises a mechanical resistance meter (12) which at least partly determines the position of the penetration means (8) with respect to the object to be treated by measuring the mechanical resistance of the penetration means (8) in the object to be penetrated.

8. Apparatus (1) according to one of the preceding claims, wherein the positioning device (5) is provided with a gear rack (5a) connected to the extendable part (4) in the longitudinal direction, which engages with a gear wheel drive (5b) which is placed in the apparatus body (2) and is connected to an operating member which can be operated by an operator.

9. Apparatus (1) according to one of the preceding claims, wherein a light source (18) which can be directed at the holder (7) is attached to the apparatus body (2) or the extendable part (4).

10. Holder (7), suitable for exchangeable connection with the extendable part (4) of an apparatus (1) according to one of the preceding claims, provided on a holder part (16) with penetration means (8) attached thereto, comprising needles, which are arranged in a pattern on the holder part (16), wherein a feature of the holder (7) can be manipulated in such a manner during use by a manipulating element (19a, 19b) arranged on the apparatus body (2) or the extendable part (4), that the holder (7) is made unsuitable for re-use in the apparatus (1), and wherein the holder comprises 10-20 needles and the needles have a length of approximately 15 mm.

11. Holder (7) according to Claim 10, wherein the holder (7) can be at least partly mechanically deformed by, during use, engaging with the manipulating element (19a, 19b) which is installed on the extendable part (4) and which comprises a mechanical deforming means (19a).

12. Holder (7) according to Claim 10, wherein the holder (7) comprises an electronic memory (20).

13. Holder (7) according to one of Claims 10-12, wherein the length of at least a part of the penetration means (8) near the circumference of the holder part (16) is smaller than the length of the other penetration means (8) situated on the holder part (16).

14. Holder (7) according to one of Claims 10-13, wherein the holder part (16) is at least partly convex on the side facing the object to be treated.

15. Holder (7) according to one of Claims 10-14, wherein one or more penetration means (8) are provided with a liquid inlet duct (15).

## Patentansprüche

1. Vorrichtung (1) zum Behandeln eines Tennisellbogens mittels perkutaner Intervention, wobei die Vorrichtung Folgendes aufweist:
- einen Vorrichtungskörper (2),
- ein Befestigungsteil (3), welches zur fixierbaren Positionierung des Vorrichtungskörpers (2) in Bezug zu seiner Umgebung in der Nähe einer äußeren Seite des Vorrichtungskörpers (2) angeordnet ist,
- ein Teil (4), welches innerhalb oder in der Nähe des Vorrichtungskörpers (2) angeordnet ist, und welches in Bezug zu dem Vorrichtungskörper (2) herausgefahren werden kann,
- eine Positionierungseinrichtung (5), welche innerhalb oder in der Nähe des Vorrichtungskörpers (2) angeordnet ist um den herausfahrbaren Teil (4) in Bezug zum Vorrichtungskörper (2) zu positionieren,
- eine Halterung (7), welche während der Benutzung zu dem herausfahrbaren Teil (4) austauschbar verbunden ist, wobei die Halterung (7) ein Halterungsteil (16) mit daran befestigen Eindring-Elementen (8), welche Nadeln aufweisen, aufweist, und wobei die Eindring-Elemente (8) in einem Muster auf dem Halterungsteil (16) angeordnet sind,
- der Vorrichtungskörper (2) oder das herausfahrbare Teil (4) ein Manipulationselement (19a, 19b) aufweist, welches einen Teil der Halterung (7) während der Benutzung derart manipuliert, dass die Halterung (7) zur Wiederbenutzung in der Vorrichtung 1 unbrauchbar gemacht wird, wobei die Positionierungseinrichtung (5) dazu geeignet ist, den herausfahrbaren Teil (4) in Bezug zu dem Vorrichtungskörper (2) genau zu positionieren um die gewünschte Eindringtiefe zum Durchführen einer perkutanen Intervention einer Ellbogensehne zu erreichen, und wobei die Halterung (7) 10-20 Nadeln aufweist, welche eine Länge von ungefähr 15 mm haben.

2. Vorrichtung (1) nach Anspruch 1, wobei die Vorrichtung (1) außerdem ein Sperr-Element (22) aufweist, welches die Vorrichtung(1) blockiert wenn eine Halterung (7) installiert worden ist, welche zur Wiederverwendung unbrauchbar gemacht wurde.

3. Vorrichtung (1) nach Anspruch 1 oder 2, wobei das Manipulationselement (19a, 19b) eine mechanische Deformationseinrichtung aufweist, welche während der Benutzung in die Halterung (7) eingreift und diese zumindest zum Teil deformiert.

4. Vorrichtung (1) nach Anspruch 1 oder 2, wobei die Halterung (7) einen elektronischen Speicher (20) aufweist und die Vorrichtung (1) eine Leseeinrichtung (25) zum Lesen des Speichers (20) aufweist, wobei das Manipulationselement (19a, 19b) eine elektronische Schreibeinrichtung (19b) aufweist, um während des Betriebes einen Wert in den elektronischen Speicher (20) zu schreiben.

5. Vorrichtung (1) nach Anspruch 4, wobei die Vorrichtung (1) eine AuswertungsSchaltung (21) aufweist, welche während der Benutzung den aus dem elektronischen Speicher (20) gelesenen Speicherwert auswertet.

6. Vorrichtung (1) nach einem der vorhergehenden Ansprüche, wobei die Vorrichtung (1) einen Sensor (11, 11') aufweist, welcher die Position der Eindring-Elemente (8) im Bezug zu dem Objekt, das behandelt werden soll, bestimmt, wobei der Sensor (11, 11') die Position der Eindring-Elemente (8) in Bezug zu dem Objekt, das behandelt werden soll, detektieren kann und die Position an die Positionierungseinrichtung (5) übermittelt, wenn ein Grenzwert in Bezug zu der Position der Eindring-Elemente (8) bezüglich des Objekts, das behandelt werden soll, überschritten wurde.

7. Vorrichtung (1) nach Anspruch 6, wobei der Sensor (11, 11') ein Messgerät (12) für den mechanischen Widerstand aufweist, welches zumindest teilweise die Position der Eindring-Elemente (8) in Bezug zu dem Objekt, das behandelt werden soll, bestimmt, indem es den mechanischen Widerstand der Eindring-Elemente (8) in dem Objekt, in das eingedrungen werden soll, misst.

8. Vorrichtung (1) nach einem der vorhergehenden Ansprüche, wobei die Positionierungseinrichtung (5) eine Zahnstange (5a) aufweist, welche mit dem herausfahrbaren Teil (4) in longitudinaler Richtung verbunden ist, wobei die Zahnstange (5a) in einen Zahnradantrieb (5b) eingreift, welcher in dem Vorrichtungskörper (2) vorgesehen ist und mit einem Betätigungselement verbunden ist, das von einem Benutzer betätigt werden kann.

9. Vorrichtung (1) nach einem der vorhergehenden Ansprüche, wobei eine Lichtquelle (18) an dem Vorrichtungskörper (2) oder dem herausfahrbaren Teil (4) angebracht ist, wobei die Lichtquelle (18) auf die Halterung (7) gerichtet werden kann.

10. Halterung (7), welche geeignet ist eine austauschbare Verbindung mit dem herausfahrbaren Teil (4) der Vorrichtung (1) nach einem der vorhergehenden Ansprüche herzustellen, wobei die Halterung (7) an einem Halterungsteil (16) angebracht ist, an dem Eindring-Elemente (8), welche Nadeln aufweisen, angebracht sind, wobei die Eindring-Elemente (8) in einem Muster auf dem Halterungsteil (16) angeordnet sind, wobei ein Bestandteil der Halterung (7) während der Benutzung derartig von einem Manipulationselement (19a, 19b), welches auf dem Vorrichtungskörper (2) oder dem herausfahrbaren Teil (4) vorgesehen ist, manipuliert werden kann, dass die Halterung (7) zur Wiederverwendung in der Vorrichtung (1) unbrauchbar gemacht werden kann, und wobei die Halterung (7) 10-20 Nadeln aufweist, welche eine Länge von ungefähr 15 mm haben.

11. Halterung (7) nach Anspruch 10, wobei die Halterung (7) zumindest teilweise während der Benutzung deformiert werden kann, wobei dies durch Eingreifen mit dem Manipulationselement (19a, 19b), welches auf dem herausfahrbaren Teil (4) installiert ist und welches eine mechanische Deformations-Einrichtung (19a) aufweist, geschehen kann.

12. Halterung (7) nach Anspruch 10, wobei die Halterung (7) einen elektronischen Speicher (20) aufweist.

13. Halterung (7) nach einem der Ansprüche 10 bis 12, wobei die Länge zumindest eines Teils der Eindring-Elemente (8) in der Nähe des Umfangs des Halterungsteils (16) kürzer ist als die Länge der anderen Eindring-Elemente (8), welche auf dem Halterungsteil (16) vorgesehen sind.

14. Halterung (7) nach einem der Ansprüche 10 bis 13, wobei das Halterungsteil (16) zumindest zum Teil konvex auf der Seite ist, welche dem Objekt das behandelt werden soll, zugewandt ist.

15. Halterung (7) nach einem der Ansprüche 10 bis 14, wobei eines oder mehrere der Eindring-Elemente (8) einen Flüssigkeits-Einlasskanal (15) aufweist bzw. aufweisen.

## Revendications

1. Appareil (1) pour le traitement d'une épicondylite latérale au moyen d'une intervention percutanée, comprenant :
un corps d'appareil (2),
une partie de fixation (3) qui est agencée à proximité d'un côté extérieur du corps d'appareil (2) pour positionner de manière fixe le corps d'appareil (2) par rapport à son environnement,
une partie (4) qui est agencée dans le corps d'appareil (2) ou à proximité de celui-ci et qui peut être étendue par rapport au corps d'appareil (2),
un dispositif de positionnement (5) qui est agencé dans le corps d'appareil (2) ou à proximité de celui-ci pour positionner la partie extensible (4) par rapport au corps d'appareil (2),
un support (7) qui est relié de manière interchangeable à la partie extensible (4) pendant l'utilisation, comprenant une partie de support (16) avec des moyens de pénétration (8) fixés à celle-ci, comprenant des aiguilles, dans lequel les moyens de pénétration (8) sont agencés sous forme d'un motif sur la partie de support (16),
dans lequel le corps d'appareil (2) ou la partie extensible (4) est pourvu(e) d'un élément de manipulation (19a, 19b) qui manipule une caractéristique du support (7) de telle sorte que, pendant l'utilisation, le support (7) soit rendu inapte pour une réutilisation dans l'appareil (1),
dans lequel le dispositif de positionnement (5) est adapté pour positionner de manière précise la partie extensible (4) par rapport au corps d'appareil (2) afin d'obtenir la profondeur de pénétration souhaitée pour réaliser une intervention percutanée d'un tendon de coude, et dans lequel le support comprend entre 10-20 aiguilles et les aiguilles ont une longueur d'environ 15 mm.

2. Appareil (1) selon la revendication 1, pourvu en outre d'un élément d'inactivation (22) qui bloque l'appareil (1) si un support (7) qui a été rendu inapte pour une réutilisation, a été installé.

3. Appareil (1) selon la revendication 1 ou 2, dans lequel l'élément de manipulation (19a, 19b) comprend un moyen de déformation mécanique qui s'engage avec le support (7) pendant l'utilisation et déforme au moins partiellement ce dernier.

4. Appareil (1) selon la revendication 1 ou 2, dans lequel le support (7) est pourvu d'une mémoire électronique (20) et l'appareil (1) est pourvu d'un dispositif de lecture (25) pour lire la mémoire (20), dans lequel l'élément de manipulation (19a, 19b) comprend un moyen d'écriture électronique (19b) pour écrire une valeur dans la mémoire électronique (20) pendant l'utilisation.

5. Appareil (1) selon la revendication 4, dans lequel l'appareil (1) est pourvu d'un circuit d'évaluation (21) pour l'évaluation, pendant l'utilisation, de la valeur de mémoire lue à partir de la mémoire électronique (20).

6. Appareil (1) selon l'une des revendications précédentes, dans lequel ce dernier est pourvu d'un capteur (11 ; 11') pour déterminer la position des moyens de pénétration (8) par rapport à l'objet devant être traité, dans lequel le capteur (11 ; 11') peut détecter la position des moyens de pénétration (8) par rapport à l'objet devant être traité et communique la position au dispositif de positionnement (5) si une valeur seuil concernant la position des moyens de pénétration (8) par rapport à l'objet devant être traité a été dépassée.

7. Appareil (1) selon la revendication 6, dans lequel le capteur (11 ; 11') comprend un dispositif de mesure de résistance mécanique (12) qui détermine, au moins partiellement, la position des moyens de pénétration (8) par rapport à l'objet devant être traité en mesurant la résistance mécanique des moyens de pénétration (8) dans l'objet devant être pénétré.

8. Appareil (1) selon l'une des revendications précédentes, dans lequel le dispositif de positionnement (5) est pourvu d'une crémaillère (5a) reliée à la partie extensible (4) dans la direction longitudinale, qui s'engage avec un entraînement par engrenage (5b) qui est placé dans le corps d'appareil (2) et est relié à un élément d'actionnement qui peut être actionné par un opérateur.

9. Appareil (1) selon l'une des revendications précédentes, dans lequel une source de lumière (18) qui peut être dirigée vers le support (7) est fixée au corps d'appareil (2) ou à la partie extensible (4).

10. Support (7), adapté pour être relié de manière interchangeable à la partie extensible (4) d'un appareil (1) selon l'une des revendications précédentes, pourvu, sur une partie de support (16), de moyens de pénétration (8) fixés à celle-ci, comprenant des aiguilles, qui sont agencées sous forme d'un motif sur la partie de support (16), dans lequel une caractéristique du support (7) peut être manipulée par un élément de manipulation (19a, 19b) agencé sur le corps d'appareil (2) ou la partie extensible (4) de sorte que, pendant l'utilisation, le support (7) soit rendu inapte pour une réutilisation dans l'appareil (1), et dans lequel le support comprend entre 10-20 aiguilles et les aiguilles ont une longueur d'environ 15 mm.

11. Support (7) selon la revendication 10, dans lequel le support (7) peut être déformé mécaniquement au moins partiellement en s'engageant, pendant l'utilisation, avec l'élément de manipulation (19a, 19b) qui est installé sur la partie extensible (4) et qui comprend un moyen de déformation mécanique (19a).

12. Support (7) selon la revendication 10, dans lequel le support (7) comprend une mémoire électronique (20).

13. Support (7) selon l'une des revendications 10 à 12, dans lequel la longueur d'au moins une partie des moyens de pénétration (8) à proximité de la circonférence de la partie de support (16) est inférieure à la longueur des autres moyens de pénétration (8) situés sur la partie support (16).

14. Support (7) selon l'une des revendications 10 à 13, dans lequel la partie de support (16) est au moins partiellement convexe sur le côté faisant face à l'objet devant être traité.

15. Support (7) selon l'une des revendications 10 à 14, dans lequel un ou plusieurs moyen(s) de pénétration (8) sont pourvu(s) d'un conduit d'entrée de liquide (15).
